# EUROPÄISCHE PATENTSCHRIFT

(11) **EP 1 879 631 B1**
(45) Veröffentlichungstag und Bekanntmachung des Hinweises auf die Patenterteilung: **02.05.2012**
(21) Anmeldenummer: 06753528.6
(22) Anmeldetag: 09.05.2006
(51) Int. Cl.: A61L 24/06, A61L 27/16, A61L 24/00, A61L 27/50

(54) **BIOAKTIVER KNOCHENZEMENT UND VERFAHREN ZU SEINER HERSTELLUNG**
BIOACTIVE BONE CEMENT AND METHOD FOR THE PRODUCTION THEREOF
CIMENT OSSEUX BIOACTIF ET PROCEDE DE FABRICATION

(30) Priorität: 13.05.2005 DE 102005023094
(43) Veröffentlichungstag der Anmeldung: 23.01.2008
(73) Patentinhaber: InnoTERE GmbH, 01307 Dresden (DE)
(72) Erfinder: NIES, Berthold, 64407 Fränkisch Crumbach (DE)
(74) Vertreter: Kailuweit & Uhlemann Patentanwälte
(86) Internationale Anmeldenummer: PCT/EP2006/004330
(87) Internationale Veröffentlichungsnummer: WO 2006/122678

(56) Entgegenhaltungen:
- EP-A1- 0 425 200
- EP-A2- 0 115 410
- WO-A-96/39107
- DE-A1- 2 905 878

## Beschreibung

Aufgabe der vorliegenden Erfindung war es, einen neuen Knochenzement, basierend auf Polymethyl-methacrylat (PMMA), Co-Polymeren und analogen Systemen, die über radikalische Polymerisation aushärten, bereitzustellen, wobei diese Zusätze enthalten, die zu einer Mineralisation der Zementoberfläche nach Inkubation in simulierter Körperflüssigkeit führen und bei denen die erhaltenen Mineralisationsschichten in ihrer Zusammensetzung Calcium Phosphat Phasen enthalten, so dass nach der Implantation in Knochen die Ausbildung fibröser Zwischenschichten unterdrückt wird.

Auf Polymeren basierende Knochenzemente sind an sich bekannt und werden beispielsweise in der Orthopädie, Unfallchirurgie und/oder Wirbelsäulenchirurgie oder auch in der Mund-, Kiefer- und Gesichtschirurgie zur Auffüllung und Überbrückung von Knochendefekten und zur Fixierung von Implantaten eingesetzt. Ihr Vorteil gegenüber anderen üblichen Materialien, wie z. B. Metallimplantaten, mineralischen Knochenzementen auf der Basis von Calcium-Phosphaten, Calcium-Phosphat-basierten Knochenersatzmaterialien und alternativen Behandlungsmethoden besteht in der einfachen Handhabung, der schnell erreichbaren Endfestigkeit (10 - 30 min.), der hohen Dauerlastfestigkeit und Beständigkeit, der relativ guten Verträglichkeit (ausreichenden Biokompatibilität), der freien Modellierbarkeit und der vergleichsweise kostengünstigen Anwendungsmöglichkeit in vielen Bereichen der Knochenchirurgie allgemein. Angesichts des hohen Qualitätsstandes dieser Materialien, die seit mehr als 40 Jahren im klinischen Einsatz sind, sind in den letzten Jahren auf dem Gebiet der polymer-basierten Knochenzemente nur wenige innovative Ansätze in die klinische Praxis eingeführt worden. Als Beispiele für aktuelle Forschungsansätze seien folgende Arbeitsrichtungen genannt:
- Verbesserung der Handhabung durch Ersatz der Pulver-FlüssigkeitsMischungen durch 2-Pasten-Systeme ;
   o (Belkoff et al; Biomechanical Evaluation of a New Bone Cement for Use in Vertebroplaty. Spine. 25(9): 1061-64, May1, 2000.)
- Verstärkung durch Faserzusatz;
   o (Saha S.; Pal S. Improvement of mechanical properties of acrylic bone cement by fibre reinforcement. J. Biomech. 17:467-478. 1984;
   o Gilbert et al. Self-Reinforced composite poly(methyl methacrylate): static and fatige properties. Biomaterials. 16:1043-1055. 1955.
- Alternative Röntgenkontrast-Medien ;
   o Van Hooy-Corstjens et al. Mechanical behaviour of a new acrylic radiopaque iodine-containing bone cement. Biomaterials, 25, 2657-2667, (2004);
   o Kjellsson et al. Tensile properties of a bone cement containing non-ionic contrast media. J Mater Sci Mater Med. 2001 Oct-Dec: 12 (10-12):889-94
- Zumischung unterschiedlicher Füll- und Trägerstoffe ;
   o Liebendörfer et al. Experimental studies on a new bone cement: hydroxyapatite composite resin. The 21st Annual Meeting of the Society for Biomaterials. San Francisco. USA, 335. 1995.
   o Shinzato et al.: Bioactive bone cement: effect of phosphoric ester monomer on mechanical properties and osteoconductivity in J. Biomed. Mater. Res. 2001; 56(4); 571-577.
   o Miyazaki et al.: Bioactive PMMA bone cement prepared by modification with methacryloxypropyltrimethoxysilane and calcium chloride. J. Biomed. Mater. Res. 2003; 67A(4); 1417-1423.
   o Fujita et al.: Bioactive bone cement: effect of the amount of glass-ceramic powder on bone bonding strength. J Biomed Mater Res.1998 Apr;(1):145-52
EP0115410 A offenbart offenbart eine Zusammensetzung mit guter Haftung an Hartgewebe wie z.B. Zähnen und Knochen. Die Zusammensetzung kann auch zum Füllen und zur Reparatur von Hartgewebe verwendet werden und enthält ein polymerisierbares, Phosphatgruppen tragendes Monomer und ein Vinylmonomer.
EP0425200 A offenbart einen Knochenzement auf Basis von Polyalkylmethacrylat, welcher 4-(2-Methacryloyl-oxyethyl)trimellitinsäure (4-MET) enthält.
WO96/391 A offenbart einen Knochenzement auf Basis von Polymethylmethacrylat mit einem Zusatz von Calciumalendronat.

Publizierte Ansätze zur Bioaktivierung von Knochenzementen basieren ausschließlich auf der Zumischung bioaktiver Substanzen zur Polymermatrix unter Verwendung meist sehr hoher Füllungsgrade (Komposit-Zemente). Dagegen wurden die Aspekte Biokompatibilität und Bioaktivität/ Osteokonduktivität von Polymer-basierten konventionellen Knochenzementen selbst bisher kaum beachtet, denn die bisher verwendeten Produkte sind zwar grundsätzlich bioverträglich, und verursachen keine ausgeprägten Fremdkörperreaktionen, haben aber den großen und eindeutigen Nachteil, dass sie nicht hinreichend bioaktiv sind, um eine direkte Anbindung an den Knochen, also eine Verwachsung mit demselben zu ermöglichen. Von einer solchen Osteokonduktivität kann man definitionsgemäß nur dann sprechen, wenn der Knochen das implantierte Material aktiv integriert und direkt, also ohne eine fibröse Zwischenschicht auszubilden, an der Oberfläche anwächst bzw. diese ohne Ausbildung eines störenden Zwischenraums belegt. Diese fibrösen Zwischenschichten, treten bei allen bisher bekannten polymer-basierten (konventionellen) Knochenzementen aufgrund der unzureichenden Integration auf. Die fibrösen, bindegewebsartigen Zwischenschichten kann man auch als Narbengewebe ansehen, über die der Körper sich nach einer Verletzung von der Umgebung oder einem Fremdkörper abgrenzt. Derartige Schichtensysteme, Knochen - fibröse Zwischenschicht - implantiertes Material, haben den großen Nachteil, dass sie mechanisch nicht stark belastbar sind und bilden die Ursache für Mikrobewegungen, die letztlich zur Abstoßung des Implantats, also zu sogenanntem Implantatversagen führen können.

Erfolgreiche Implantationen mit Polymer-basierten Knochenzementen sind daher von einer innigen Verzahnung von spongiösem Knochen und der pastösen Zementmasse während der Implantation stark abhängig. Gerade diese Notwendigkeit schränkt das Einsatzgebiet von Polymer-basierten Knochenzementen erheblich ein. Dieser Nachteil wiegt um so schwerer, als viele alternative und konkurrierende Implantatmaterialien inzwischen mit osteokonduktiven Oberflächen ausgestattet sind, z. B. Metallimplantate mit bioaktiven Beschichtungen, mineralische Knochenzemente auf der Basis von Calcium-phosphaten, Calcium-phosphat-basierte Knochenersatzmaterialien.

Abgrenzung zum Stand der Technik:
Die Patentrecherche zu bioaktiven PMMA-Zementen (PMMA = Polymethylmethacrylat) brachte keine Treffer bzw. Fundstellen. In der Literatur werden bioaktive PMMA-Zemente ausschließlich als Komposite aus PMMA-Zement und Füllstoffen aus bioaktivem Glas oder Hydroxylapatit beschrieben.

Von besonderem Interesse ist in diesem Zusammenhang die Publikation von Shinzato et al.: Bioactive bone cement: effect of phosphoric ester monomer on mechanical properties and osteoconductivity in J. Biomed. Mater. Res. 2001; 56(4); 571-577. Anders als in der (weiter unten beschriebenen) vorliegenden Erfindung wird in diesem Beispiel von Shinzato das Phosphorsäure-Ester-Monomer aber auch hier nicht einem klassischen PMMA-Zement zugesetzt, sondern wird als Adhäsionsvermittler zu einem PMMA-Bioglas-Komposit-Zement beigefügt. Der Effekt auf die Mechanik und die Bioaktivität wird als positiv beschrieben. Die Autoren interpretieren die gefundenen Ergebnisse als Effekt der im Vergleich zu MMA (MMA = Methylmetacrylat) geringeren Polymerisationsneigung des Phosphorsäure-Ester (PE) -Monomers, die letztlich zu einer Anreicherung bzw. stärkeren Exposition der Bioglas-Partikel an der Zementoberfläche führt. Ein Hinweis auf die bioaktive Wirkung des PE-Monomers und anderer erfindungsgemäßer Monomere in klassischen PMMA-Zement ist dieser Publikation nicht zu entnehmen. Die bioaktive Wirkung wird ausschließlich den Bioglas-Partikeln zugeschrieben.

In der Arbeit von Miyazaki et al.: Bioactive PMMA bone cement prepared by modification with methacryloxypropyltrimethoxysilane and calcium chloride (J. Biomed. Mater. Res. 2003; 67A(4); 1417-1423) wird die Bildung von Apatit auf entsprechend modifizierten Zementen nach Inkubation in SBF (SBF = Simulated Body Fluid) beschrieben. Die als notwendig angegebenen Konzentrationen sind allerdings so hoch, dass sowohl das Abbindeverhalten, als auch die mechanischen Eigenschaften des erhaltenen Zements signifikant verschlechtert werden.

Beide Publikationen lassen die in unseren Versuchen zur vorliegenden Erfindung gefundenen Ergebnisse nicht erwarten und legen diese auch nicht nahe, zumal in der Arbeit von Shinzato die Bioaktivität dem Zusatz von ca. 70 Gewichts-% an bioaktivem Glas zugeschrieben wird. In der Arbeit von Miyazaki wird eine Apatit-Bildung auf der Zementoberfläche nur nach Zusatz von mehr als 16% CaCl₂ festgestellt. In unseren Versuchen zur vorliegenden Erfindung dagegen, wird schon bei geringen Zusätzen an erfindungsgemäßen Monomeren, wie z. B. Methacrylsäure oder Ethylenglycol-Methacrylat-Phosphat, bei Anteilen von weniger als 10% Gewichtprozent, vorzugsweise aber unter 5% Gewichtsprozent und besonders bevorzugt unter 3 Gewichtsprozent, ohne Zusatz von CaCl₂ der gewünschte Effekt der Bioakvität festgestellt und nachgewiesen. In unserer erfindungsgemäßen Problemlösung zur Bereitstellung eines verbesserten bioaktiven Knochenzements liegt die primäre Wirkung bei der Bildung von Kristallisationskeimen, die durch spontane Calcium-Ionen Freisetzung aus dem Knochenzement zugemischten wasserlöslichen Calciumsalzen und kurzfristige Anhebung des lokalen pH-Werts auf neutrale bis leicht alkalische Werte unterstützt wird. Dieser überraschende und ebenso unerwartete Effekt wird bei keinem bisher bekannten Knochenzemente bzw. Polymer-basierten Knochenzemente beobachtet.

Das einzige derzeit bekannte, am Markt verfügbare Produkt eines Polymer-basierten Knochenzements, das den Anspruch erhebt, bioaktiv zu sein, ist Cortoss® von Orthovita. Hierbei handelt es sich um ein Kompositmaterial aus einer vernetzenden Polymermatrix mit einem hohen Füllstoffgehalt an partikulärem Bioglas. Dort wo die Bioglas-Partikel an die Oberfläche des Zements zu liegen kommen, kommt die Bioaktivität des Bioglases zum tragen. Die Polymermatrix selbst ist auch in diesem Fall, im Gegensatz zur vorliegenden Erfindung, nicht bioaktiv. Der notwendigerweise hohe Zuschlag an bioaktivem Füllstoff von bis zu 70 Gewichtsprozent ist bei diesem Zement - ebenso wie bei einigen aus anderen Quellen bekannten experimentellen Zusammensetzungen (s.o.) - mit erheblichen Nachteilen hinsichtlich relevanter, unbedingt benötigter Zementeigenschaften für eine Reihe klinisch bedeutender Indikationen verbunden. Besonders ins Gewicht fallen veränderte mechanische Daten und hier insbesondere eine stark erhöhte Steifigkeit (E-Modul) bei geringerer Biegefestigkeit. Aufgrund dieser Sprödigkeit ist Cortoss® nicht für die Befestigung von Gelenkimplantaten geeignet. Weitere Nachteile ergeben sich aus der Notwendigkeit, diese Art von Zementen als 2-Pasten-Systeme auslegen zu müssen, da sich die Feststoff- und Flüssigkeits-Komponenten nicht in konventioneller Weise mischen lassen. Besonders hervorzuheben sind hier die resultierenden Probleme der Sedimentation des Bioglas-Füllstoffs und der regelmäßig stattfindende, vorzeitige Zerfall des Radikalstarters vor der Aushärtung (während der Lagerung). Beide Probleme begrenzen die Lagerstabilität erheblich und machen eine dauerhafte Lagerung im Kühlschrank notwendig. Als Polymer-Komponente kommen bei diesem Zement vernetzende Makromere auf der Basis von Bisphenol A (Bis-GMA = Bisphenol A Glycidyl Methacrylat) zum Einsatz, die potenziell eine höhere Toxizität aufweisen als das konventionelle Methyl-Methacrylat. Ein weiterer großer Nachteil solcher Zemente auf der Basis neuer Polymer-Basis-Zusammensetzungen (für das Einsatzgebiet als Implantatmaterialien) ist die mangelnde Langzeiterfahrung im Vergleich mit den bislang eingesetzten Produkten aus der Gruppe der konventionellen PMMA-Knochenzemente, die aber allesamt nicht bioaktiv sind.

Die Aufgabe und das Ziel der vorliegenden Erfindung ist es daher, einen Weg zur Erzielung einer bioaktiven/osteokonduktiven Zementoberfläche zu finden, die sich nach oder während der Anmischung und Implantation schnell aber auch dauerhaft ausbildet, und dabei die anderen relevanten Eigenschaften des Ausgangszements beibehält, ohne diese, wie im Fall von Cortoss® zuvor beschrieben, nachteilig zu beeinflussen. Es ist dem Fachmann bekannt, dass zweckmäßigerweise die Menge an zugesetzten Komponenten zur Erzielung der Bioaktivität möglichst gering zu halten ist. Der verfolgte Forschungsansatz war daher gegensätzlich zu solchen 2-Pasten-Systemen wie Cortoss®, die im Sinne der Bioaktivität die Zusammensetzung in einer Weise modifizieren müssen, dass über einen sehr hohen Füllungsgrad ausreichend viele bioaktive Partikel an die Implantatoberfläche kommen.

Ein bekanntes Prinzip zur Bioaktivierung von Materialoberflächen im Knochenkontakt ist die Beschichtung oder sonstige Erzeugung von Calcium Phosphat Phasen auf der Materialoberfläche, insbesondere bei Metallen (HA-Plasma-Spray oder elektrochemisch gestützte Beschichtung - BoneMaster). Versuche zur Bioaktivierung mittels Abscheidung von Calciumphosphat-Phasen auf Polymeren wurden bei einer Reihe von Materialien synthetischen und biologischen Ursprungs durchgeführt:
- Mineralisierung von Gelatine, R. Kniep, S. Busch, Angew. Chem., 1996,108,2788
- Mineralisierung von Kollagen, S. Rössler et al. Mineralised collagen coating as a biomimetic approach to implant surfaces. Biomaterials 2004.
- Kokubo et al. Apatite formation on non-woven fabric of carboxymethylated chitin in SBF. Biomaterials, 2003.
- Kawai et al. Coating of an apatite layer on polyamide films containing sulfonic groups by a biomimetic process. Biomaterials, 2003

Nachteilig für eine mögliche technische/medizinische Anwendung war bei den bekannten synthetischen Polymeren aber immer, sofern sie überhaupt als lasttragende Implantatmaterialien in Betracht kommen können, dass sie entweder chemisch vorbehandelt werden mussten, um saure Gruppen als Kristallisationskeime an der Oberfläche zu erhalten und/oder die Notwendigkeit erfordern, wasserlösliche Calcium Salze einzuarbeiten, wie im Beispiel von Miyazaki et al. zuvor beschrieben, wo eine Apatit-Bildung auf der Zementoberfläche nur nach Zusatz von mehr als 16% CaCl₂ erreicht werden kann. Nach diesen Modifikationen zeigten auch die synthetischen Polymere eine mehr oder weniger gute Mineralisierung mit Calcium Phosphat Phasen nach Inkubation in simulierter Körperflüssigkeit wie z. B. SBF (simulated body fluid, Rezeptur siehe unten), die darauf schließen lässt, dass sich entsprechende Mineralphasen nach der Implantation auch in vivo ausbilden können.

Zementartige Präparationen auf Polymerbasis mit dem Potenzial zur Oberflächen-Mineralisierung in SBF sind aus der Literatur bisher nicht bekannt, offenbar auch aufgrund der Tatsache, dass die bekannten Ansätze bei Zementen nicht anwendbar sind, da im Gegensatz zu fertig geformten Implantaten sich die Oberfläche eines Zements erst im Verlauf der Anmischung, bzw. während und nach der Einbringung in den Körper bildet. Es ist zwingend erforderlich und eine Grundlage der vorliegenden Erfindung, das Ansätze zur Bioaktivierung der Zementoberfläche daher ebenfalls im Zuge der Zementaushärtung an der Oberfläche wirksam werden müssen. Die eigentliche Mineralisierung kann dann vorzugsweise in vivo erfolgen, sie sollte allerdings möglichst schnell vonstatten gehen, um den Knochenzellen in der Umgebung bereits früh nach der Implantation die Möglichkeit zur Adhäsion zu geben.

Im Sinne der Aufgaben- und Zielstellung der vorliegenden Erfindung wurde überraschend gefunden, dass der Zusatz von geringen Mengen an polymerisierbaren Monomeren, die anionische Gruppen enthalten, die Oberflächeneigenschaften von Polymer-basierten Knochenzementen in einer Weise beeinflussen, dass Proben aus entsprechend modifizierten Knochenzementen nach Inkubation in SBF spontan mit einer Schicht aus Calcium-Phosphat Phasen bedeckt werden. Der erforderliche Zusatz an Monomeren mit anionischen Gruppen zu ansonsten unveränderten Polymer-basierten Knochenzementen ist von der Auswahl des jeweiligen Monomers abhängig, liegt aber in der Regel unter 10 Gewichtsprozent bezogen auf die Gesamtmasse der Zementpräparation und bevorzugt unter 5% bezogen auf die Gesamtmasse. Besonders bevorzugt sind Zumischungen im Konzentrationsbereich zwischen 0,03% und 3% bezogen auf die Zementmasse. Es können auch Monomerenmischungen eingesetzt werden, bevorzugt sind aber reine Monomere.

Einfachstes erfindungsgemäßes Monomer ist die Methacrylsäure, weitere sind Acrylamidoglycolsäure, Ethylenglycol-Methacrylat-Phosphat, Sulfopropyl-Methacrylat, 2-Acrylamido-2-Methylpropan-Sulfonsäure. Bei diesen Beispielen handelt es sich um ausgewählte Monomere aus verschiedenen Gruppen. Im weiteren Verlauf wird das Prinzip der Erfindung und die daraus abgeleitete Auswahl der Zusätze detailliert beschrieben, so dass dem Experten klar wird, welche Zusätze als erfindungsgemäß anzusehen sind, auch wenn sie nicht explizit genannt sind.

Der gefundene Effekt bestätigt, dass entsprechend modifizierte konventionelle Polymer-basierte Knochenzemente, bzw. entsprechend ausgerüstete neuartige Zementzusammensetzungen von Polymer-basierten Knochenzementen eine hohe Bioaktivität nach Implantation in den Körper aufweisen, indem sie sich mit einer Calcium Phosphat Schicht überziehen und dadurch osteokonduktiv werden. Diese, mit der vorliegenden Erfindung erzielte Verbesserung, versetzt uns also in die Lage, Polymer-basierte Knochenzemente herzustellen, die osteokonduktiv sind und sich fest - ohne nachteiliger Ausbildung fibröser Zwischenschichten oder anderer Zwischenräume - mit dem Knochengewebe verbinden können.

Gegenstand der Erfindung ist daher ein Knochenzemente auf der Basis von Polymethyl-methacrylat (PMMA), Co-Polymeren und analogen Systemen, die über radikalische Polymerisation aushärten, aber dadurch gekennzeichnet sind, dass sie Zusätze enthalten, die zu einer Mineralisation der Zementoberfläche nach Inkubation in simulierter Körperflüssigkeit führen und bei denen die erhaltenen Mineralisationsschichten in ihrer Zusammensetzung Calcium Phosphat Phasen enthalten, so dass nach der Implantation in Knochen die Ausbildung fibröser Zwischenschichten unterdrückt wird.

Weiterhin kennzeichnend für die erfindungsgemäßen Knochenzemente sind die gebildeten Mineralablagerungen, die in simulierter Körperflüssigkeit zu größer 80% aus präzipitiertem Hydroxylapatit bzw. Calcium-defizientem und/oder substituiertem, carbonatisiertem und /oder Na-, K-, oder Mgsubstituiertem Hydroxylapatit bestehen. Die Knochenzemente enthalten einen Zusatz (nachfolgend Zusatz 1), der als Mineralisationskeim für die heterogene Keimbildung für die Abscheidung von Mineralisationsschichten und insbesondere Calcium Phosphat Phasen dienen kann.

Gegenstand der Erfindung ist außerdem ein Knochenzement, bei dem der Zusatz 1 jeweils mindestens eine polymerisierbare Monomereinheit, wie z. B. Acrylat-, Methacrylat-, Vinyl-, oder sonstige ethylenisch ungesättigte Doppelbindung enthält oder dieser aus Co-Oligomeren oder Co-Polymeren besteht, die unter Verwendung der zuvor genannten Monomere hergestellt wurden.

Erfindungsgemäße Monomere (als Zusatz zu Polymer-basierten Knochenzementen) enthalten einerseits mindestens eine ethylenisch, ungesättigte Doppelbindung, über die das Monomer-Molekül bei radikalischer Polymerisation in die Polymermatrix des Knochenzements eingebaut werden kann und andererseits mindestens eine anionische Gruppe, die nach erfolgter Polymerisation an der Zementoberfläche als Kristallisations-keim für die Mineralisierung in vivo dienen kann. Beide Gruppen können über chemisch unterschiedlich zusammengesetzte Molekülabschnitte miteinander verbunden sein.

Die polymerisierbare Funktionalität des Monomers kann unterschiedlich zusammengesetzt sein und darf im wesentlichen eine oder mehrere Gruppen aufweisen, die zu einer radikalischen Polymerisation geeignet sind und die sich direkt oder über einen Spacer mit einer anionischen Gruppe modifizieren lassen. Die radikalische Polymerisation kann hierbei vorzugsweise unter Umgebungsbedingungen erfolgen, kann aber auch durch Wärm oder Lichtquellen etc. initiiert werden. Weiterhin kann die polymerisierbare Funktionalität des Monomers eine oder mehrere olefinisch ungesättigte Doppelbindungen enthalten, ohne den Gegenstand und Sinn der Erfindung zu verändern. Bevorzugte Gruppen für diesen Abschnitt sind Acrylat- und Methacrylat-Gruppen, aber auch Vinyl- und Styrol-Derivate. Ausdrücklich eingeschlossen sind Monomere, die mehr als eine Methacrylat- bzw. Vinyl- und/oder Styrol-Gruppe enthalten.

Alternativ zur kovalenten Anbindung erfindungsgemäßer Zusätze zum Knochenzement können diese auch über Nebenvalenzen an die Zementmatrix angebunden sein. Die polymerisierbare Funktionalität kann daher auch von Molekülabschnitten ersetzt werden, die eine gute Verträglichkeit mit der Zementmatrix aufweisen und sich aus diesem Grund nachhaltig mit ihr verbinden. Entsprechende Molekülabschnitte können daher aus grundsätzlich allen Zusammensetzungen gebildet sein, für die diese Voraussetzung zutrifft. Besonders in Betracht kommen Komponenten, die mit der Polymermatrix des Zements verwandt sind. Als Beispiele seien hier vor allem Oligomere und Polymere des (Meth-)Acrylats, Vinyls oder Styrols, sowie deren Co-Oligomere und Co-Polymere untereinander und mit anderen radikalisch polymerisierbaren Monomeren genannt, so wie sie aus der Polymer-Industrie bekannt sind. Als erfindungsgemäße Verbindungen enthalten diese mindestens jeweils eine direkt oder über Spacer angebundene Funktionalität, die zu einer anionischen Gruppe dissoziieren oder hydrolysieren kann.

Als Spacer fungiert eine optional verwendeter oder notwendiger Molekülabschnitt, über die eine polymerisierbare Gruppe mit der anionischen Gruppe verbunden ist/wird. Dabei kann der Spacer praktisch jede Zusammensetzung aufweisen, die dem Sinn der Erfindung nicht widerspricht. Insbesondere kann der Spacer großen Einfluss auf die Kompatibilität (z. B. Löslichkeit und Polymerisationsgeschwindigkeit) des zugesetzten Monomers mit der sonstigen Zementflüssigkeit haben. Vorausgesetzt wird, dass der Spacer selbst und seine Verbindung mit den anderen Funktionalitäten (dem polymerisierbaren Molekülabschnitt und der anionischen Gruppe) unter Lagerbedingungen chemisch stabil und physiologisch unbedenklich sind. Für den Spacer kommen vor allem verzweigte oder unverzweigte Kohlenwasserstoffe mit 1-18 C-Atomen, kurzkettige Poly-Ether, kurzkettige Poly-Ester (jeweils 1-12 Einheiten, z.B. PEG und PPG) Poly-Aminosäuren (z.B. Poly-Amino-Hexansäure), aromatische Verbindungen mit einem oder mehreren Benzolringen und ähnliche in Betracht.

Erfindungsbestimmend ist vor allem der Molekülabschnitt des Monomer-Zusatzes, der unter physiologischen Bedingungen in eine anionische Gruppe dissoziieren oder hydrolysieren kann (anionische Funktionalität), da er letztlich für die Ausbildung von Kristallisationskeimen auf der Zementoberfläche entscheidend ist. Experimentell wurde in dieser Erfindung der erfindungsgemäße Effekt anhand zahlreicher Beispiele für Monomere mit Carboxyl-, Phosphat-, Posphonat- und Sulfatgruppen nachgewiesen (siehe Beispiele). Die anionische Funktionalität kann jedoch auch jeweils mehr als eine der zuvor genannten Gruppen enthalten, also Mischungen von Carboxyl-, Phosphat-, Posphonat-, Sulfatgruppen und/oder anderen geeigneten anionischen Gruppen enthalten, ohne den Sinn der Erfindung zu verändern oder den beobachteten Effekt zu ändern. Neben den Monomeren mit freien, dissoziierbaren, anionischen Gruppen sind auch solche Verbindungen eingeschlossen, die erst nach der Implantation in den Körper und bei Kontakt mit wässriger Lösung zu dissoziierbaren, anionischen Gruppen hydrolysieren, vorzugsweise und insbesondere Ester der Carboxylgruppe, Phosphat-, Phosphonat- und Sulfatgruppen. Beispiele für geeignete Monomere (Zusätze):
- **Carboxylgruppen**-haltig: Einfachstes erfindungsgemäßes Monomer ist die Methacrylsäure (die keinen Spacer enthält); Acrylamidoglycolsäure
- **Phosphatgruppen**-haltig: Ethylenglycol-Methacrylat-Phosphat; Homologe mit mehr als einer Ethylenglycol-Einheit
- **Phosphonatgruppen**-haltig: Ethylenglycol-Methacrylat-Phosphonat; Homologe mit mehr als einer Ethylenglycol-Einheit
- **Sulfatgruppen**-haltig: Sulfopropyl-Methacrylat; 2-Acrylamido-2-Methylpropan-Sulfonsäure

Die genannten Beispiele für den Zusatz 1 sollen die möglichen Ausgestaltungsformen der Erfindung anhand einfacher Lösungen demonstrieren; sie sollen aber in keiner Weise als Einschränkung angesehen werden. Komplexer aufgebaute Zusätze, die mehr als eine der oben genannten Funktionalitäten tragen und/oder weitere Funktionalitäten enthalten, die in der obigen Beschreibung nicht genannt sind (beispielsweise Funktionalitäten, die aus biologischen Molekülen abgeleitet sind), sind ausdrücklich als erfindungsgemäße Zusätze anzusehen, sofern sie sich mit der Zementmatrix verbinden oder an dieser adhärieren und nach Inkubation von Zementproben, die diese Zusätze enthalten, in simulierter Körperflüssigkeit (SBF) zur Ausbildung von Mineralisation auf der Zementoberfläche führen.

Gegenstand der Erfindung sind zudem auch Knochenzemente, bei denen der Zusatz 1 eine Funktionalität aufweist, über die er während der Aushärtung oder Polymerisationsreaktion in die Zementmatrix eingebunden oder an diese adsorbiert werden kann und bei denen der Zusatz 1 eine Funktionalität aufweist, die unter physiologischen Bedingungen in eine anionische Gruppe dissoziieren oder hydrolysiert werden kann und bei dem diese beiden Funktionalitäten direkt oder über einen Spacer-Abschnitt miteinander verbunden sind. Der Zusatz 1 liegt vorzugsweise in einer Endkonzentration von 0,01 bis 10 Gewichts-%, besonders bevorzugt bei 0,01 bis 5 Gewichts-%, in der Gesamtmasse des abgebundenen Knochenzements vor. Der am meisten bevorzugte Konzentrationsbereich liegt zwischen 0,03 und 3 Gewichts% des abgebundenen Knochenzements.

Eine bevorzugte Ausführungsform besteht darin, dass der Knochenzement so vorbereitet angeboten wird, dass der Zusatz mit der Monomerflüssigkeit schon homogen vermischt oder in der Monomerflüssigkeit bereits gelöst ist oder bei denen der Zusatz in fester Form fein verteilt und homogen mit dem Zementpulver vermischt ist.

Zur Verstärkung des erfindungsgemäßen Effekts können dem Polymer-basierten Knochenzement weitere Zusätze beigemischt werden, um die Mineralisierungsneigung der Oberfläche nach der Implantation zu erhöhen. Dies ist optional möglich, muss aber nicht erfolgen und gilt somit als besondere Ausführungsform der Erfindung.

Als besonders wirksam werden wasserlösliche Calcium Salze als Zusatz (Zusatz 2) angesehen, wie auch experimentell nachgewiesen (siehe Beispiele). Die Freisetzung von Ca²+-Ionen aus der oberflächennahen Zementmatrix erhöht lokal die Ca²+-Konzentration in der Nähe des Zements und führt zu einer schnelleren und stärkeren Ausbildung von Calcium Phosphat Phasen an den Kristallisationskeimen. Bevorzugt sind bioverträgliche Calcium-Salze, deren Löslichkeit in Wasser vorzugsweise bei über 1 g/l liegt. Beispiele sind: CaCl₂, Ca(NO₃)₂, Calciumacetat, Calciumascorbat oder ein anderes Calcium-Salz einer im tierischen Organismus natürlich vorkommenden organische Säure oder eine Mischung derartiger Salze ist. Die Zumischung dieser Salze (Zusatz 2) liegt im Bereich von 0,01 bis 20 Gewichtsprozent, bevorzugt zwischen 0,1 und 10 Gewichtsprozent. Besonders bevorzugt sind jedoch Zumischungen an wasserlöslichen Calcium-Salzen im Bereich zwischen 0,1 und 7,5 Gewichtsprozenten.

Weitere erfindungsgemäße Zusätze sind Puffersubstanzen (Zusatz 3), die durch ihre Freisetzung an der Zementoberfläche zu einer Anhebung des lokalen pH-Wertes führen. Aufgrund der pH-Wert-abhängigen Löslichkeit und Kristallisation von Calcium Phosphat Phasen führt eine Anhebung des lokalen pH-Wertes zu einer stärkeren Zunahme der relativen Übersättigung bezüglich der Bildung von Calcium Phosphat Phasen im Vergleich zum Löslichkeitsprodukt an der Zementoberfläche und zu einer Förderung der Abscheidung von Hydroxylapatit (bzw. Ca-defizientem und carbonatisiertem Hydroxylapatit). Als pH-Wert anhebende Puffersubstanzen (Zusatz 3) kommen hauptsächlich und vorzugsweise Na₂CO₃, NaHCO₃, Na₃PO₄, Na₂HPO₄, Na₃Citrat in Betracht (bzw. die entsprechenden K-Salze) - grundsätzlich aber alle bioverträglichen Puffersubstanzen, deren größte Pufferkapazität im neutralen oder leicht basischen Bereich liegt vorzugsweise aber solche, deren pK-Wert bei oder höher als 7,4 liegt. Die Zumischung der Puffersubstanzen liegt im Bereich zwischen 0,1 und 15 Gewichtsprozent, bevorzugt bei 0,1 bis 10 Gewichtsprozent bezogen auf die Gesamtmasse des Zements. Besonders bevorzugt sind Zumischungen an Puffersubstanz im Bereich zwischen 0,1 und 7,5 Gewichtsprozent.

Die Gesamtmenge an erfindungsgemäßen Zumischungen (Zusätze 1 bis 3, ohne Röntgenkontrast und Antibiotika) liegt bevorzugt unter 20 Gewichtsprozent bezogen auf die Gesamtmasse und besonders bevorzugt unter 10 Gewichtsprozent, wobei, besonders bevorzugt, der Gehalt an Zusatz 1 0,03 bis 3 Gewichtsprozent, der Gehalt an Zusatz 2 0,1 bis 7,5 Gewichtsprozent und der Gehalt an Zusatz 3 0,1 bis 7,5 Gewichtsprozent beträgt.

Gegenstand der Erfindung sind ferner Knochenzemente, bei denen die erfindungsgemäßen Zusätze und sonstigen Zementkomponenten in 2- oder Mehr-Pastensystemen formuliert sind und dort entweder suspendiert oder gelöst in der Polymerpaste vorliegen.

Außerdem können die erfindungsgemäßen Knochenzemente noch weitere Zusätze, wie z. B. Röntgenkontrastmittel, Antibiotika, andere antimikrobielle Wirkstoffe und/oder anti-inflammatorische Wirkstoffe enthalten, die in der Lage sind Entzündungsreaktionen des Körpers nach der Implantation des Zements zu unterdrücken.

Weiterhin sind Bestandteil der Erfindung, Knochenzementformulierungen, die in geschlossenen oder teilweise geschlossenen Mischsystemen formuliert sind und/oder die als fertig konfektionierte Systeme in sterilisierter Form vorliegen. Ebenso umschließt die Erfindung Knochenzementformulierungen, die als Bausatz aus zwei oder mehr getrennt abgepackten und in ihren Mengenverhältnissen aufeinander abgestimmten Komponenten bestehen, die erst unmittelbar vor der Anwendung miteinander kombiniert und gemischt werden.

Schließlich umfasst die Erfindung die Verwendung des erfindungsgemäßen Knochenzements zur Verankerung von Prothesenkomponenten im Knochen, zur Versteifung von Knochen, zur Füllung und Rekonstruktion von Knochendefekten aller Art, als Dübel für Knochenschrauben oder als Implantatmaterial zur Verankerung von Schrauben u.a. Implantaten für die Osteosynthese.

Der erfinderische Ansatz und dessen Lösung des aus dem Stand der Technik bekannten Problems, geht aus den nachfolgenden Darstellungen, Abbildungen und Beispielen näher hervor. Die nachfolgenden Darstellungen, Abbildungen und Beispiele sollen die Erfindung näher erläutern, diese aber keinesfalls einschränken.

Beispiele:
Allgemeine Versuchsbeschreibung:
   Aus handelsüblichem Knochenzement - Palacos R^{®}, (Biomet Merck) - wurden Probekörper der Dimension 2 mm Dicke und 10 mm Durchmesser hergestellt, indem der Knochenzement nach Angaben des Herstellers aus Pulver und Flüssigkeit im Verhältnis 2:1 (Gewicht zu Volumen) angemischt wurde. Die erhaltene Zementpaste wurde auf befeuchteten Glasplatten in Rahmen aus Polyethylen ausgestrichen, deren Öffnung und Dicke der Probendimension entsprach. In dieser Form wurden die Proben bei Umgebungsbedingungen ausgehärtet und anschließend entnommen (entformt). In den nachfolgenden Untersuchungen wurde jeweils die während der Aushärtung an der befeuchteten Glasplatte anliegende Probenfläche ausgewertet. Als Kontrolle diente jeweils unmodifizierter Knochenzement - Palacos R^{®} von Biomet Merck. Die experimentellen Zusammensetzungen unterschieden sich von der Kontrolle durch Zusätze von erfindungsgemäßen Monomeren zur Zementflüssigkeit (Beispiele 1+2) bzw. durch Zusatz von löslichen Calcium Salzen und Puffersubstanzen zum Zementpulver bei gleichzeitigem Einsatz von anionischem Monomer zur Zementflüssigkeit (Beispiele 3+4).

Im Anschluss an die Herstellung wurden die Proben in 1,5-fach konzentrierter SBF bei 37°C inkubiert. Die verwendete SBF hatte folgende Zusammensetzung: 150 mmol/l NaCl; 4,2 mmol/l NaHCO₃; 1,5 mmol/l MgCl₂; 1 mmol/l K₂HPO₄; 5 mmol/l KCI; 2,4 mmol/l CaCl₂; pH=7,4. Die Auswertung erfolgte mittels eines Scores zur Auswertung der Mineralisierung der Probenoberfläche anhand Rasterelektronenmikroskopischer Aufnahmen. Zusätzlich wurden die Probenoberflächen physikalisch charakterisiert und die chemische Zusammensetzung der Mineralschicht und deren Phasenzusammensetzung durch Röntgenbeugungsanalyse bestimmt.

Mineralisierungs-Score:
Die Angaben bezeichnen das Ausmaß der Mineralisierung in den Stufen 0 bis 3, wobei 0 keine Mineralisierung, 1 vereinzelte, 2 fortgeschrittene und 3 vollständige Mineralisierung, nach der jeweiligen Inkubationszeit in Stunden, bedeuten. Also bedeutet z. B. 2/24 eine fortgeschrittene Mineralisierung nach einer Inkubationszeit von 24 Stunden.

**Tabelle 1**

| Mineralisierungseffekt in Zeitabhängigkeit | | | | |
|---|---|---|---|---|
| **Mineralisierung/Zeit** | **1h** | **24h** | **72h** | **168h** |
| **keine M. (0)** | 0/1 | 0/24 | 0/72 | 0/168 |
| **vereinzelte M. (1)** | 1/1 | 1/24 | 1/72 | 1/168 |
| **fortgeschrittene M. (2)** | 2/1 | 2/24 | 2/72 | 2/168 |
| **Vollständige und kristalline M. (3)** | 3/1 | 3/24 | 3/72 | 3/168 |

### Beispiel 1

Knochenzement (Palacos R) mit x% Methacrylsäure-Zusatz (MAA) zum Monomer; keine weiteren Zusätze.

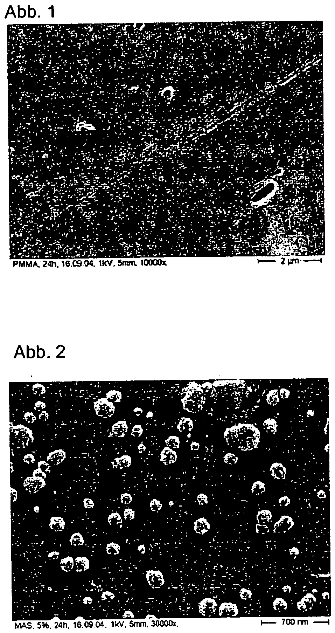

Ergebnis:
- 0% MAA: 0/1, 0/24(Abb. 1), 0/72, 0/168 Vergleichsversuch (s. Abb. 1)
- 0,5% MAA: 1/24,
- 1 % MAA: 1/24,
- 2,5% MAA: 1/24,
- 5,0% MAA: 1/24, (s Abb. 2)

Durch Zusatz von MAA wird eine relativ geringe Mineralisierung erreicht, die zudem nur eine geringe Konzentrationsabhängigkeit aufweist.

### Beispiel 2

Knochenzement (Palacos R) mit x% Ethylenglycol-Methacrylat-Phosphat (HEMA-P) zum Monomer; keine weiteren Zusätze.

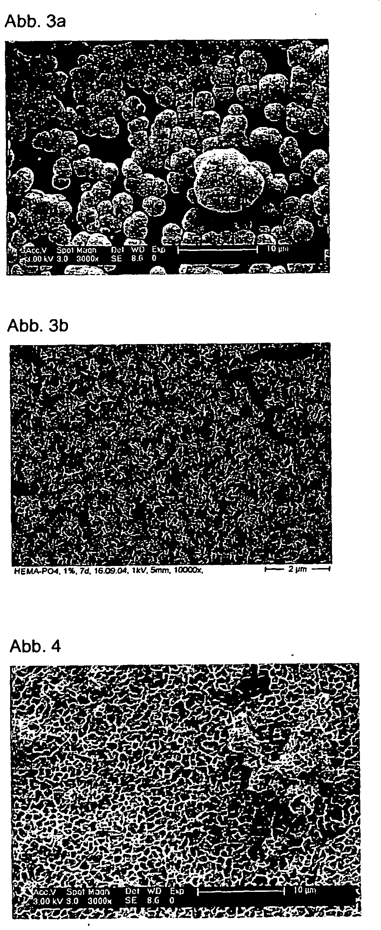

Ergebnis:
- 0,5% HEMA-P: 1/24,
- 1% HEMA-P: 1/1, 1/24, 2/72 (s. Abb.3a), 3/168 (s. Abb. 3b)
- 2,5% HEMA-P: 2/24,
- 5% HEMA-P: 2/24 (dichte Schicht)

Durch Zusatz von HEMA-P wird bereits bei niedriger Konzentration eine deutliche Mineralisierung erzielt, die mit steigender Konzentration weiter zunimmt. Mit steigender Inkubationsdauer nimmt ebenfalls die Kristallinität der Mineralschicht zu.

### Beispiel 3

Knochenzement (Palacos R) mit a) 1 % HEMA-P und b) 2,5% MAA im Monomer und jeweils 2% CaCl₂ im Pulver; keine weiteren Zusätze.
a) Verstärkte Mineralisierung und früherer Übergang zu einer kristallinen Form; Score: 3/24 (Abb.:4; vergleichbar mit 5% HEMA-P aus Beispiel 2).
b) 1/24; Mineralisierung ebenfalls tendenziell verstärkt; Unterschied weniger deutlich als bei HEMA-P

### Beispiel 4:

Knochenzement (Palacos R^{®}) mit 1% HEMA-P im Monomer und 5% Na₂CO₃ im Pulver; keine weiteren Zusätze.

Ergebnis: Tendenziell Verstärkung der Mineralisierung und frühere Erreichung des kristallinen Zustandes.

(die qualitative Bewertung lässt keine feinere Differenzierung des Mineralisierungsgrades zu).

## Patentansprüche

1. Polymer-basierter Knochenzement, der über radikalische Polymerisation aushärtet, **dadurch gekennzeichnet, dass** der Knochenzement einen Zusatz 1 sowie mindestens einen weiteren Zusatz ausgewählt aus Zusatz 2 und / oder Zusatz 3 enthält, wobei die Zusätze zu einer Mineralisation der Zementoberfläche nach Inkubation in simulierter Körperflüssigkeit führen und die erhaltenen Mineralisationsschichten in ihrer Zusammensetzung Calcium-Phosphat-Phasen enthalten, die die Ausbildung fibröser Zwischenschichten nach der Implantation in den Körper unterdrücken, wobei
- Zusatz 1 Monomere mit freien, dissoziierbaren oder hydrolysierbaren anionischen Gruppen enthält,
- Zusatz 2 wasserlösliche Calcium-Salze enthält,
- Zusatz 3 bioverträgliche Puffersubstanzen, deren größte Pufferkapazität im neutralen oder leicht basischen Bereich liegt, enthält.

2. Knochenzement nach Anspruch 1, **dadurch gekennzeichnet, dass** in Zusatz 1 die freien, dissoziierbaren oder hydrolysierbaren anionischen Gruppen Carboxyl-, Phosphat-, Phosphonat- oder Sulfatgruppen oder deren bioverträgliche Ester sind.

3. Knochenzement nach Anspruch 2, **dadurch gekennzeichnet, dass** Zusatz 1 mindestens eine polymerisierbare Monomereinheit enthält oder aus Co-Oligomeren oder Co-Polymeren besteht, die unter Verwendung der Monomeren nach Anspruch 2 hergestellt werden.

4. Knochenzement nach Anspruch 3, **dadurch gekennzeichnet, dass** Zusatz 1 als Monomer Methacrylsäure, Acrylamidoglycolsäure, Ethylenglycol-Methacrylat-Phosphat oder dessen Homologe mit mehr als einer Ethylenglycol-Einheit, Ethylenglycol-Methacrylat-Phosphonat oder dessen Homologe mit mehr als einer Ethylenglycol-Einheit, Sulfopropyl-Methacrylat oder 2-Acrylamido-2-Methylpropan-Sulfonsäure enthält.

5. Knochenzement nach Anspruch 1, **dadurch gekennzeichnet, dass** die Löslichkeit der Calcium-Salze in Zusatz 2 in Wasser bei über 1 g/l liegt.

6. Knochenzement nach Anspruch 5, **dadurch gekennzeichnet, dass** das Calcium-Salz in Zusatz 2 CaCl₂, Ca(NO₃)₂, Ca-(Acetat)₂, Ca-(Ascorbat)₂ oder ein anderes Calciumsalz einer im tierischen Organismus natürlich vorkommenden organische Säure oder eine Mischung derartiger Salze ist.

7. Knochenzement nach Anspruch 1, **dadurch gekennzeichnet, dass** die bioverträgliche Puffersubstanz in Zusatz 3 Na₂CO₃, NaHCO₃, Na₃PO₄, Na₂HPO₄, Na₃-Citrat bzw. ein entsprechendes Kalium-Salz ist.

8. Knochenzement nach Anspruch 1, **dadurch gekennzeichnet, dass** der Zusatz 1 mit der Monomerflüssigkeit homogen vermischt oder in der Monomerflüssigkeit gelöst ist oder der Zusatz 1 fein verteilt und homogen mit dem Zementpulver vermischt ist.

9. Knochenzement nach Anspruch 1, **dadurch gekennzeichnet, dass** der Zusatz 1 in einer Endkonzentration von 0,01 bis 10 Gewichtsprozent in der Gesamtmasse des abgebundenen Knochenzements vorliegt.

10. Knochenzement nach Anspruch 1, **dadurch gekennzeichnet, dass** der Zusatz 2 in einer Endkonzentration von 0,01 bis 20 Gewichtsprozent bezogen auf die wasserfreie Substanz vorliegt.

11. Knochenzemente nach Anspruch 1, **dadurch gekennzeichnet, dass** der Zusatz 3 in einer Endkonzentration von 0,1 bis 15 Gewichtsprozent bezogen auf die Gesamtmasse des Zements vorliegt.

12. Knochenzement nach Anspruch 1, **dadurch gekennzeichnet, dass** die Summe der Gewichtsprozente sämtlicher Zusätze 1 bis 3 weniger als 20 Gewichtsprozent der Endkonzentration bezogen auf die Gesamtmasse ausmacht und der Gehalt an Zusatz 1 0,03 bis 10 Gewichtsprozent , der Gehalt an Zusatz 2 0,1 bis 7,5 Gewichtsprozent und der Gehalt an Zusatz 3 0,1 bis 7,5 Gewichtsprozent beträgt.

13. Knochenzement nach Anspruch 1, **dadurch gekennzeichnet, dass** die enhaltenen Zusätze in 2- oder Mehr-Pastensystemen formuliert sind und dort entweder suspendiert oder gelöst in der Polymerpaste vorliegen.

14. Knochenzement nach Anspruch 1, **dadurch gekennzeichnet, dass** weitere Zusätze ausgewählt aus einem Röntgenkontrastmittel, Antibiotika, antimikrobiellen Wirkstoffen und antiinflammatorischen Wirkstoffen enthalten sind.

15. Knochenzement nach Anspruch 1, **dadurch gekennzeichnet, dass** der Knochenzement in einem geschlossenen oder teilweise geschlossenen Mischsystemen formuliert ist und/oder als fertig konfektioniertes System in sterilisierter Form vorliegt.

16. Knochenzement nach Anspruch 1, **dadurch gekennzeichnet, dass** der Knochenzement als Bausatz aus zwei oder mehr getrennt abgepackten und in ihren Mengenverhältnissen aufeinander abgestimmten Komponenten besteht, die erst unmittelbar vor der Anwendung miteinander kombiniert werden.

17. Knochenzement nach Anspruch 1 zur Anwendung bei der Verankerung von Prothesenkomponenten im Knochen, zur Versteifung von Knochen, zur Füllung und Rekonstruktion von Knochendefekten aller Art, als Dübel für Knochenschrauben oder als Implantatmaterial zur Verankerung von Schrauben u.a. Implantaten für die Osteosynthese.

## Claims

1. Polymer-based bone cement that cures by radical polymerization, **characterized in that** the bone cement comprises an additive 1 and at least one further additive selected from additive 2 and/or additive 3, wherein the additives cause the cement surface to mineralize upon incubation in simulated body fluid and in which the obtained mineral layers contain calcium phosphate phases that prevent the formation of fibrous intermediate layers after implantation into the body, wherein
- additive 1 comprises monomers with free, dissociatable or hydrolyzable anionic groups,
- additive 2 comprises water-soluble calcium salts,
- additive 3 comprises biocompatible buffering substances with the highest buffering capacity thereof being in the neutral to slightly basic range.

2. Bone cement according to claim 1, **characterized by** that in additive 1 the free, dissociatable or hydrolyzable anionic groups are carboxy, phosphate, phosphonate or sulfate groups or the biocompatible esters thereof.

3. Bone cement according to claim 2, **characterized in that** additive 1 contains at least one polymerizable unit or consists of co-oligomers or co-polymers, which were produced using the monomers according to claim 2.

4. Bone cement according to claim 3, **characterized in that** additive 1 comprises as a monomer methacrylic acid, acrylamide glycolic acid, ethyleneglycol methacrylate phosphate or homologues thereof with more than one ethylene glycol unit, ethylene glycol methacrylate phosphonate or homologues thereof with more than one ethylene glycol unit, sulfopropyl methacrylate or 2-acrylamido-2-methylpropane sulfonic acid.

5. Bone cement according to claim 1, **characterized in that** the solubility of the calcium salts in additive 2 is higher than 1g/L in water.

6. Bone cement according to claim 5, **characterized in that** the calcium salt in additive 2 is CaCl₂, Ca(NO₃)₂, Ca(acetate)₂, Ca(ascorbate)₂, or another calcium salt of a naturally occurring organic acid in the organism of the animal, or a mixture of such salts.

7. Bone cement according to claim 1, **characterized in that** the biocompatible buffering substance in additive 3 is Na₂CO₃, NaHCO₃, Na₃PO₄, Na₂HPO₄, Na₃-citrate or a respective potassium salt thereof.

8. Bone cement according to claim 1, **characterized in that** additive 1 is homogeneously mixed with the monomeric liquid or is dissolved in the monomeric liquid, or **in that** additive 1 is finely and homogeneously distributed in the cement powder.

9. Bone cements according to claim 1, **characterized in that** additive 1 is contained at a final concentration of 0.01 to 10 weight percent of the total mass of the hardened bone cement.

10. Bone cement according to claim 1, **characterized in that** additive 2 is contained at a concentration of 0.1 to 20 weight percent based on the water-free substance.

11. Bone cements according to claim 1, **characterized in that** additive 3 is contained at a final concentration of 0.1 to 15 weight percent based on the water-free substance.

12. Bone cement according to claim 1, **characterized in that** the sum in weight percent of all the additives 1 to 3 does is less than 20 weight percent in the final concentration based on the total mass, wherein the content of additive 1 is 0.03 to 10 weight percent, the content of additive 2 is 0.1 to 7.5 weight percent and the content of additive 3 is 0.1 to 7.5 weight percent.

13. Bone cement according to one claim 1, **characterized in that** the contained additives are formulated in two-paste or multiple paste systems, and are contained therein either suspended or dissolved in the polymer paste.

14. Bone cement according to claim 1, **characterized in that** the bone cement comprises further additives, selected from x-ray contrast media, antibiotics, antimicrobial agents, and anti-inflammatory agents.

15. Bone cement according to claim 1, **characterized in that** the bone cement is formulated in a closed or partially closed mixing system and/or is available as ready-made and sterilized system.

16. Bone cement according to claim 1, **characterized in that** the bone cement consists of a kit of two or more components, wherein the components are separately packaged and aligned in their proportions to one another, and wherein the components are combined just before application.

17. Bone cement according to claim 1 for use in anchoring prosthesis components in the bone, for stiffening of bone, for filling and reconstruction of bone defects of all kinds, as dowels for bone screws or as implant material for anchoring of screws and other implants used for osteosynthesis.

## Revendications

1. Ostéociment à base de polymères durcissant par polymérisation radicalaire, **caractérisé par le fait que** ledit ostéociment renferme un adjuvant 1, ainsi qu'au moins un adjuvant supplémentaire choisi parmi un adjuvant 2 et/ou un adjuvant 3, lesdits adjuvants gouvernant une minéralisation de la surface du ciment après incubation dans un fluide corporel simulé, et la composition des couches de minéralisation intégrées contenant des phases calcium-phosphate qui empêchent la formation de couches intermédiaires fibreuses après l'implantation dans le corps, sachant que
- l'adjuvant 1 renferme des monomères à groupes anioniques libres, dissociables ou hydrolysables,
- l'adjuvant 2 renferme des sels de calcium hydrosolubles,
- l'adjuvant 3 renferme des substances tampons à compatibilité biologique, dont la capacité maximale de tamponnage se situe dans la plage neutre ou légèrement basique.

2. Ostéociment selon la revendication 1, **caractérisé par le fait que**, dans l'adjuvant 1, les groupes anioniques libres dissociables ou hydrolysables sont des groupes carboxyles, phosphates, phosphonates ou sulfates, voire les esters biologiquement compatibles de ces derniers.

3. Ostéociment selon la revendication 2, **caractérisé par le fait que** l'adjuvant 1 renferme au moins une unité monomère polymérisable, ou est constitué de co-oligomères ou de copolymères produits par utilisation des monomères conformes à la revendication 2.

4. Ostéociment selon la revendication 3, **caractérisé par le fait que** l'adjuvant 1 renferme, en tant que monomère, de l'acide méthacrylique, de l'acide acrylamido-glycolique, du phosphate de méthacrylate d'éthylène glycol ou ses homologues comportant plus d'une unité d'éthylène glycol, du phosphonate de méthacrylate d'éthylène glycol ou ses homologues comportant plus d'une unité d'éthylène glycol, du méthacrylate de sulfopropyle ou de l'acide sulfonique 2-acrylamido-2-méthylpropane.

5. Ostéociment selon la revendication 1, **caractérisé par le fait que** l'hydrosolubilité des sels de calcium, dans l'adjuvant 2, se situe au-delà de 1 g/l.

6. Ostéociment selon la revendication 5, **caractérisé par le fait que**, dans l'adjuvant 2, le sel de calcium est du CaCl₂, du Ca(NO₃)₂, du Ca-(acétate)₂, du Ca-(ascorbate)₂ ou un autre sel de calcium d'un acide organique naturellement présent dans l'organisme animal, voire un mélange de sels de ce type.

7. Ostéociment selon la revendication 1, **caractérisé par le fait que** la substance tampon à compatibilité biologique se présente respectivement, dans l'adjuvant 3, comme du Na₂CO₃, du NaHCO₃, du Na₃PO₄, du Na₂HPO₄, du Na₃-citrate ou un sel de potassium correspondant.

8. Ostéociment selon la revendication 1, **caractérisé par le fait que** l'adjuvant 1 est mélangé au fluide monomère de manière homogène, ou est dissous dans ledit fluide monomère, ou bien ledit adjuvant 1 est mélangé à la poudre de ciment de manière homogène et finement répartie.

9. Ostéociment selon la revendication 1, **caractérisé par le fait que** l'adjuvant 1 est présent en une concentration finale de 0,01 à 10 pour cent en poids dans la masse totale de l'ostéociment à l'issue de sa prise.

10. Ostéociment selon la revendication 1, **caractérisé par le fait que** l'adjuvant 2 est présent en une concentration finale de 0,01 à 20 pour cent en poids rapportée à la substance anhydre.

11. Ostéociment selon la revendication 1, **caractérisé par le fait que** l'adjuvant 3 est présent en une concentration finale de 0,1 à 15 pour cent en poids rapportée à la masse totale du ciment.

12. Ostéociment selon la revendication 1, **caractérisé par le fait que** la somme des pourcentages en poids de tous les adjuvants 1 à 3 représente moins de 20 pour cent en poids de la concentration finale rapportée à la masse totale, et la teneur en adjuvant 1 est comprise entre 0,03 et 10 pour cent en poids, la teneur en adjuvant 2 est comprise entre 0,1 et 7,5 pour cent en poids, et la teneur en adjuvant 3 est comprise entre 0,1 et 7,5 pour cent en poids.

13. Ostéociment selon la revendication 1, **caractérisé par le fait que** les adjuvants renfermés sont formulés en des systèmes amalgamés à 2 composants ou plus, dans lesquels ils se présentent soit en suspension, soit en solution dans l'amalgame polymère.

14. Ostéociment selon la revendication 1, **caractérisé par le fait que** d'autres adjuvants renfermés sont sélectionnés parmi un agent de contraste radiographique, des antibiotiques, des substances actives antimicrobiennes et des substances actives anti-inflammatoires.

15. Ostéociment selon la revendication 1, **caractérisé par le fait que** ledit ostéociment est formulé en un système de mélange fermé ou partiellement fermé, et/ou se présente comme un système prêt à l'emploi sous forme stérilisée.

16. Ostéociment selon la revendication 1, **caractérisé par le fait que** ledit ostéociment comprend, en tant qu'ensemble à élaborer, des composants au nombre de deux ou plus qui sont conditionnés séparément, dont les rapports quantitatifs sont réciproquement coordonnés, et dont la combinaison mutuelle ne s'opère qu'à l'instant précédant directement l'utilisation.

17. Ostéociment selon la revendication 1, dévolu à l'utilisation lors de l'ancrage d'éléments prothétiques dans l'os en vue de la rigidification osseuse, du comblement et de la reconstruction de défectuosités osseuses de toutes natures, en tant que chevilles destinées à des vis à implanter dans l'os, ou en tant que matériau d'implantation pour l'ancrage de vis, entre autres d'implants affectés à l'ostéosynthèse.
